# EUROPEAN PATENT SPECIFICATION

(11) **EP 0 598 948 B1**
(45) Date of publication and mention of the grant of the patent: **29.10.1997**
(21) Application number: 92203592.8
(22) Date of filing: 20.11.1992
(51) Int. Cl.: A61M 16/04

(54) **Neck flange for trackeostomy tube**
Flanschansatz für einen Tracheotomietubus
Bride avec collerette pour tube de trachéotomie

(43) Date of publication of application: 01.06.1994
(73) Proprietor: MALLINCKRODT MEDICAL, INC., St. Louis, Missouri 63134 (US)
(72) Inventor: Deily, Michael, Tustin, California 92680 (US); Crandall, Norman, Costa Mesa, California 92626 (US)
(74) Representative: Smulders, Theodorus A.H.J., Ir.

(56) References cited:
- GB-A- 2 007 789
- GB-A- 2 205 504
- US-A- 4 304 228
- US-A- 5 054 482
- US-A- 5 056 515

## Description

This invention relates to a neck flange for a tracheostomy tube to position and support the tracheostomy tube when inserted into the neck of a human wherein the neck flange contains a flat sheet-like neck engaging portion made of a flexible polymer material and possessing a centrally located aperture and an interconnection made of a polymer material that is less flexible than the polymer material of the neck engaging portion, having a ring-shaped body with an opening therethrough occupying a space that is common to the aperture, and secured to the neck engaging portion, carried as part thereof, the interconnection being connectable to a tracheostomy tube passing through the opening.

Such a neck flange, known from US-A-4,304,228, comprises a neck flange with a bushing and an annular collar. The bushing is secured to the neck engaging portion by suitable means such as, for example, an adhesive, whereas the annular collar is rotatably mounted on the bushing. The tracheostomy tube is divided in two parts, the one part being unified with the neck engaging portion and the other part being connectable to said neck engaging portion by said annular collar resulting in a generally non-movable connection between the neck flange and the tracheostomy tube.

US-A-5,056,515 discloses a neck flange with a neck engaging portion movably connectable to a tracheostomy tube by an interconnection in the shape of two pivotal tabs integrally formed with said neck engaging portion, i.e. the interconnection and the neck engaging portion are made of the same (flexible or less flexible) material.

The invention provides a neck flange according to the preamble of claim 1, the interconnection of which has a pair of opposed pivot pins extending inwardly into the opening towards each other and adapted to carry movably the tracheostomy tube, said ring-shaped body being encapsulated in the polymer of the neck engaging portion with the pair of opposed pivot pins remaining not encapsulated. In such a neck flange all parts meant for engaging the neck of the patient can be made of comfortable flexibly conforming material, whereas the interconnection is made of less flexible material. This is achieved by encapsulating the less flexible material by the flexible material of the neck engaging portion leaving only the pivot pins not encapsulated.

Preferably, the interconnection is designed to permit limited swivel motion of the tracheostomy tube relative to the opening and substantially within a plane generally normal to the flat sheet of the neck engaging portion. Further, it is preferred that the interconnection has a stepped cross section so that the pair of opposed pivot pins are raised relative to one of the major surfaces of the ring-shaped body of the interconnection. In case according to a further embodiment of the invention the neck engaging portion and the interconnection are substantially transparent, it is possible for medical practitioners to observe entry of the tube into the neck of the patient. The interconnection can be molded of a polymer having a hardness of approximately 70-100 Shore D per ASTM D-785 and the hardness of the neck engaging portion can be in the range of approximately 85-90 Shore A per ASTM D-2240.

The invention further relates to a method for manufacturing a neck flange having a flat sheet-like neck engaging portion of a flexible material and a ring-shaped interconnection of a less flexible material being separately molded from a polymer and then secured to the neck engaging portion, as known from US-A-4,304,228. According to the present invention it is proposed that the ring-shaped interconnection is, during molding, provided with a pair of opposed pivot pins extending inwardly into a central opening of the ring-shaped interconnection, and, after molding, inserted into a mold for molding the neck engaging portion, whereafter polymer is injected in the mold to form the neck engaging portion about the ring-shaped interconnection such that of the ring-shaped interconnection all but the pair of opposed pivot pins is encapsulated in the flat sheet-like neck engaging portion. By overmolding, with exception of the pivot pins, the interconnection made of a less flexible material by the more flexible neck engaging portion, the result is a neck flange that can be used together with a tracheostomy tube having a distal end to be placed in the neck of a human to be a passage and aid for breathing, a proximal end to be positioned outside the neck of the human and a central part between its ends, wherein the central part includes recesses on opposite sides thereof across from one another, the pair of opposed pivot pins being arranged to be fit for movement into said recesses.

With reference to the accompanying drawings a preferred embodiment of the neck flange according to the invention will now be further discussed and elucidated.

Figure 1 is a perspective view of a neck flange of the preferred embodiment shown with a tracheostomy tube in position against and within the patient's neck and, for clarity, part of the neck and neck flange is sectioned away.

Figure 2 is a front end view of the preferred neck flange and again the tracheostomy tube is shown, including a referenced inner cannula and cuff with its inflation tube.

Figure 3 is a side view in cross section of the neck flange of Figure 2 as would be seen if the cross section were taken along line 3-3 in Figure 2 and the tracheostomy tube were not included.

Figure 4 is a side view in cross section of the neck flange of Figure 3 or as would be seen if the cross section were taken along line 4-4 in Figure 3 and the tracheostomy tube were not included.

Figure 5 is front view of the interconnection that is less flexible than the neck engaging portion and insert molded within the neck engaging portion.

The invention has as its objectives an improvement in a neck flange system for a tracheostomy tube that includes a relatively flexible neck engaging portion and a relatively rigid interconnection from which the tracheostomy tube is positioned and methods of its manufacture. The invention is not to be construed as limited to the structure for the neck flange described and illustrated by way of example and the methods of manufacture specifically explained.

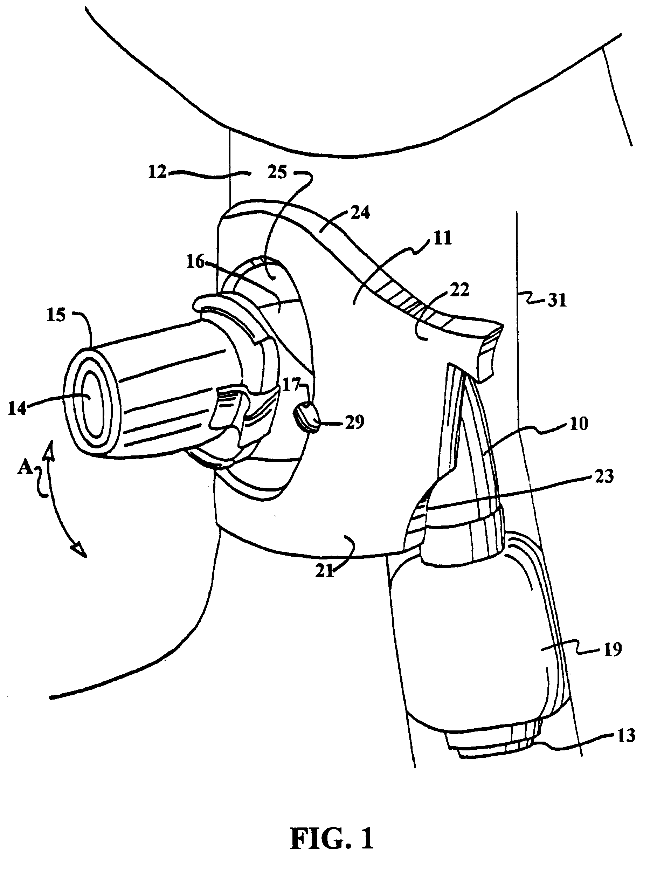

Figure 1 is a perspective view of a tracheostomy tube 10 and neck flange 11 of the preferred embodiment shown with the tracheostomy tube 10 in position against and within the patient's neck 12. For clarity of illustration, part of the neck 12 is shown removed. The tracheostomy tube 10 and neck flange 11 may be used with a tracheostomy procedure on the neck 12 of a human. The tracheostomy tube 10 has a distal end 13 for placement in the neck 12 of the patient to form a passage 14 from the proximal end 15 open to an air source to aid breathing. The tracheostomy tube 10 includes a proximal end 15 positioned outside the human's neck 12 when the tracheostomy tube 10 is in place for use. A central part 16 is between the ends 13 and 15 of tracheostomy tube 10. The central part 16 includes recesses 17 on opposite sides thereof across from one another.

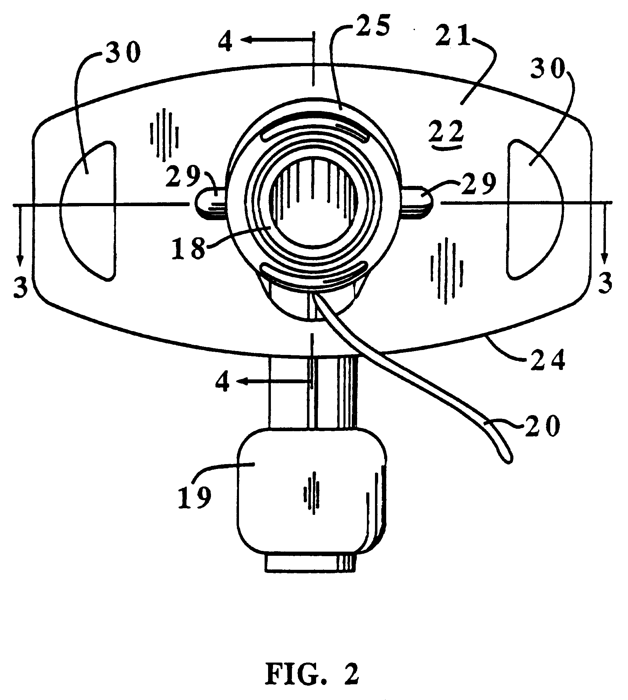

Figure 2 is a front end view of the preferred neck flange 11 and the tracheostomy tube 10 is shown including an inner cannula 18 and cuff 19 with its inflation tube 20. As can be seen in Figures 1 and 2 the neck flange 11 has a neck engaging portion 21 made of a thin flexible sheet. The sheet is preferably flat but may be curved. It contains a pair of generally parallel major surfaces 22 and 23 defined by an edge 24 around it, and a generally central aperture 25 extending through it. The neck engaging portion 21 of the preferred embodiment is molded of a flexible, transparent polymer material.

The preferred polymer material should have a hardness of less than 85 Shore A, as measured per ASTM D-2240. A hardness in the range of 70 to 85 Shore A with a minimum tensile strength of 138 x 10⁵ N/m² (2000 psi) as measured per ASTM D-638 is desired. Polymers such as polyvinylchloride, polyurethane or other flexible transparent medical grade polymers may be used for molding the neck engaging portion 21. Preferably, the particular polymer selected should have additives to maintain the relative transparency during and after sterilization by retort, gas or radiation of up to 0,035 J/kg (3.5 rads).

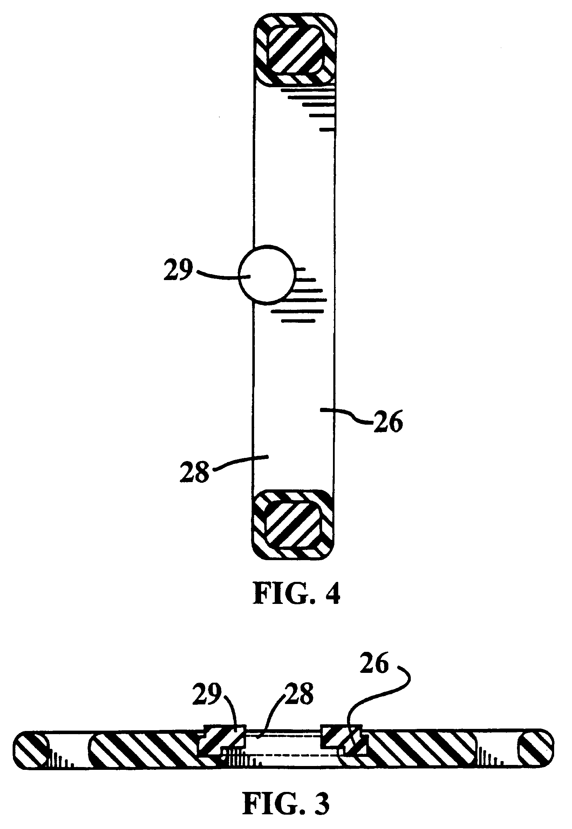

Figure 3 is a side view in cross section of the neck flange 11 of Figure 2 as would be seen if the cross section were taken along line 3-3 in Figure 2 with the tracheostomy tube 10 removed or not shown. Interconnection 26 is positioned centrally within neck engaging portion 21 and carried thereby as shown in Figure 3. Interconnection 26 has a ring-shaped body 27 containing centered opening 28 as shown in Figures 3, 4 and 5. A pair of opposed pivot pins 29 extend toward one another in a radial direction inwardly into opening 28 for supporting central part 16 of the tracheostomy tube passing therethrough, as illustrated in Figures 1 and 2.

As shown in Figure 3, interconnection 26 has a stepped cross section with the pair of opposed pivot pins 29 raised relative to its ring-shaped body 27. The pair of opposed pins 29 are arranged to be fit for movement into recesses 17 in the tracheostomy tube 10 for permitting limited swivel motion thereof as depicted by arcuate arrows A in Figure 1 and substantially within a plane generally normal to major surface 22 of the neck engaging portion.

The interconnection 26 is molded of a transparent polymer material less flexible than the neck engaging portion 21. The preferred polymer material for the interconnection 26 should have a hardness of about 85 Shore D, as measured per ASTM D-785 and an Izod notched of at least 0,0214 Nm/m (4.0 ft lb/in.) tested per ASTM D-256. Polymers, which are relatively strong, rigid and non-yielding, such as polycarbonate or other transparent medical grade polymers are useful for the interconnection 26. The polymer selected should preferably have additives to maintain the relative transparency during and after sterilization by retort, gas or radiation of up to 0,035 J/kg (3.5 rads).

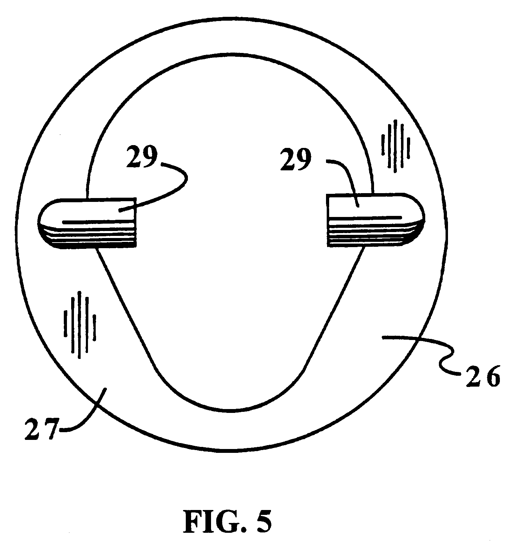

Figure 4 is a side view in cross section of the neck flange 11 of Figure 3, or as would be seen if the cross section were taken along line 4-4 in Figure 3 and the tracheostomy tube 10 were not included. Interconnection 26 is injection molded to be used as an insert in the molding of the neck engaging portion 21. The shape of interconnection 26 is shown in Figure 5. As shown there, the mold designs for the interconnection and the neck engaging portion are such that the neck engaging portion 21 leaves the raised pair of opposed pins 29 exposed. Thus, interconnection 26 is made first having the preferred shape depicted in Figure 5. Interconnection 26 is then inserted into an injection mold cavity, having the shape of the neck engaging portion 21 and also accommodating the positioning of interconnection 26. The softer, more flexible polymer is injected into that cavity to form neck engaging portion 21. The resulting neck flange 11 is a combination of materials with neck engaging portion 21 surrounding interconnection 26 with the exception of pivot pins 29. The cross sectional view of Figure 3 shows neck engaging portion 21 encapsulating the ring-shaped body of interconnection 26, and shows that pivot pins 29 are not encapsulated.

Figures 2 and 3 illustrate a pair of D shaped holes 30 in neck engaging portion 21. The pair of D shaped holes 30 are used for attachment of a neck band (not shown) which is fitted around neck 12 to hold tracheostomy tube 10 in the entry through neck 12 of the patient. Although D shaped holes 30 are preferred, any shape including oval, elliptical or square, that would conveniently attach to a neck band can be used. Each hole 30 is spaced from the areas in neck engaging portion 21 that encapsulate interconnection 26. The trached 31 is shown with an inflated cuff 19 near the distal end 13.

## Claims

1. A neck flange (11) for a tracheostomy tube (10) to position and support the tracheostomy tube (10) when inserted into the neck (12) of a human wherein the neck flange (11) contains a flat sheet-like neck engaging portion (21) made of a flexible polymer material and possessing a centrally located aperture (25) and an interconnection (26) made of a polymer material that is less flexible than the polymer material of the neck engaging portion (21), having a ring-shaped body (27) with an opening (28) therethrough occupying a space that is common to the aperture (25), and secured to the neck engaging portion (21), carried as part thereof, the interconnection (26) being connectable to a tracheostomy tube (10) passing through the opening (28), characterized in that the interconnection (26) has a pair of opposed pivot pins (29) extending inwardly into the opening (28) towards each other and adapted to carry movably the tracheostomy tube (10), said ring-shaped body (27) being encapsulated in the polymer of the neck engaging portion (21) with the pair of opposed pivot pins (29) remaining not encapsulated.

2. A neck flange according to claim 1, characterized in that the interconnection (26) permits limited swivel motion of the tracheostomy tube (10) relative to the opening (28) and substantially within a plane generally normal to the flat sheet of the neck engaging portion (21).

3. A neck flange according to claim 1 or 2, characterized in that the interconnection (26) has a stepped cross section so that the pair of opposed pivot pins (29) are raised relative to one of the major surfaces of the ring-shaped body (27) of the interconnection (26).

4. A neck flange according to any one of the preceding claims, characterized in that the neck engaging portion (21) and the interconnection (26) are molded of different polymers.

5. A neck flange according to any one of the preceding claims, characterized in that the neck engaging portion (21) and the interconnection (26) are substantially transparent.

6. A neck flange according to claim 5, characterized in that the neck engaging portion (21) and the interconnection (26) retain transparency after sterilization.

7. A neck flange according to claim 5 or 6, characterized in that the neck engaging portion (21) and the interconnection (26) are such that they remain imperceptibly less transparent after a radiation sterilization process of up to 0,035 J/kg (3.5 rads).

8. A neck flange according to any one of the preceding claims, characterized in that the interconnection (26) is molded of a polymer having a hardness greater than that of the polymer of the neck engaging portion (21).

9. A neck flange according to any one of the preceding claims, characterized in that the hardness of the interconnection (26) is 85 Shore D per ASTM D-785 so that the pair of opposed pivot pins (29) are less flexible than the neck engaging portion (21).

10. A neck flange according to any one of the claims 1-8, characterized in that the hardness of the pair of opposed pivot pins (29) is 85 Shore D per ASTM D-785.

11. A neck flange according to any one of the preceding claims, characterized in that the hardness of the neck engaging portion (21) is in the range of 70 to 85 Shore A per ASTM D-2240.

12. A method for manufacturing a neck flange (11) having a flat sheet-like neck engaging portion (21) of a flexible material and a ring-shaped interconnection (26) of a less flexible material being separately molded from a polymer and then secured to the neck engaging portion (21), characterized in that the ring-shaped interconnection (26) is, during molding, provided with a pair of opposed pivot pins (29) extending inwardly into a central opening (28) of the ring-shaped interconnection (26), and, after molding, inserted into a mold for molding the neck engaging portion (21), whereafter polymer is injected in the mold to form the neck engaging portion (21) about the ring-shaped interconnection (26) such that of the ring-shaped interconnection (26) all but the pair of opposed pivot pins (29) is encapsulated in the flat sheet-like neck engaging portion (21).

13. A method according to claim 12, characterized in that the molding process includes the steps of selecting polymers that are flexible and transparent so that the neck engaging portion (21) is more flexible than the ring-shaped interconnection (26) and the combination is transparent.

14. An assembly of a neck flange (11) according to any one of the claims 1-11 and a tracheostomy tube (10) for use in a tracheostomy procedure in the neck of a human, said tracheostomy tube (10) having a distal end (13) to be placed in the neck of a human to be a passage (14) and aid for breathing, a proximal end (15) to be positioned outside the neck of the human and a central part (16) between its ends (13, 15), characterized in that the central part (16) includes recesses (17) on opposite sides thereof across from one another, the pair of opposed pivot pins (29) being arranged to be fit for movement into said recesses (17).

## Patentansprüche

1. Halsflansch (11) für einen Tracheotomieschlauch (10) zum Positionieren und Halten des Tracheotomieschlauches (10), wenn er in einen Hals (12) eines Menschen eingeführt ist, wobei der Halsflansch (11) einen flachen folienartigen Anlageteil (21) aus einem flexiblen Polymermaterial und mit einer mittig angeordneten Apertur (25) und einer Verbindung (26) aus einem Polymermaterial aufweist, das weniger flexibel als das Polymermaterial des Halsanlageteils (21) ist, welche einen ringförmigen Körper (27) mit einer durchgehenden Öffnung (28) aufweist, die einen mit der Apertur (25) gleichen Raum einnimmt, und an dem Halsanlageteil (21) befestigt ist, wobei sie als Teil davon getragen wird, wobei die Verbindung (26) mit einem durch die Öffnung (28) gehenden Tracheotomieschlauch (10) verbindbar ist, dadurch gekennzeichnet, daß die Verbindung (26) ein Paar gegenüberliegender Lagerstifte (29) aufweist, die sich nach innen in die Öffnung (28) aufeinander zu erstrecken und geeignet sind, den Tracheotomieschlauch (10) bewegbar zu tragen, wobei der ringförmige Körper (27) in dem Polymer des Halsanlageteils (21) eingekapselt ist, wobei die gegenüberliegenden Lagerstifte (29) nicht eingekapselt bleiben.

2. Halsflansch nach Anspruch 1, dadurch gekennzeichnet, daß die Verbindung (26) eine begrenzte Schwenkbewegung des Tracheotomieschlauches (10) relativ zu der Öffnung (28) und im wesentlichen innerhalb einer generell zu der flachen Folie des Halsanlageteils (21) senkrechten Ebene ermöglicht.

3. Halsflansch nach Anspruch 1 oder 2, dadurch gekennzeichnet, daß die Verbindung (26) einen abgestuften Querschnitt aufweist, derart, daß das Paar gegenüberliegender Lagerstifte (29) aus einer der Hauptflächen des ringförmigen Körpers (27) der Verbindung (26) herausragt.

4. Halsflansch nach einem der vorhergehenden Ansprüche, dadurch gekennzeichnet, daß der Halsanlageteil (21) und die Verbindung (26) aus verschiedenen Polymeren geformt sind.

5. Halsflansch nach einem der vorhergehenden Ansprüche, dadurch gekennzeichnet, daß der Halsanlageteil (21) und die Verbindung (26) im wesentlichen transparent sind.

6. Halsflansch nach Anspruch 5, dadurch gekennzeichnet, daß der Halsanlageteil (21) und die Verbindung (26) nach der Sterilisation weiterhin transparent bleiben.

7. Halsflansch nach Anspruch 5 oder 6, dadurch gekennzeichnet, daß der Halsanlageteil (21) und die Verbindung (26) derart auagebildet sind, daß ihre Transparenz nach einem Strahlungssterilisationsvorgang von bis zu 0,035 J/kg (3,5 Rad) unmerklich abnimmt.

8. Halsflansch nach einem der vorhergehenden Ansprüche, dadurch gekennzeichnet, daß die Verbindung (26) aus einem Polymer mit einer Härte geformt ist, die größer als die des Polymers des Halsanlageteils (21) ist.

9. Halsflansch nach einem der vorhergehenden Ansprüche, dadurch gekennzeichnet, daß die Härte der Verbindung (26) 85 Shore D nach ASTM D-785 ist, derart, daß das Paar gegenüberliegender Lagerstifte (29) weniger flexibel als der Halsanlageteil (21) ist.

10. Halsflansch nach einem der Ansprüche 1-8, dadurch gekennzeichnet, daß die Härte des Paars gegenüberliegender Lagerstifte (29) 85 Shore D nach ASTM D-785 ist.

11. Halsflansch nach einem der vorhergehenden Ansprüche, dadurch gekennzeichnet, daß die Härte des Halsanlageteils (21) im Bereich von 70 bis 85 Shore D nach ASTM D-2240 liegt.

12. Verfahren zur Herstellung eines Halsflansches (11) mit einem flachen folienartigen Anlageteil (21) aus flexiblem Material und einer ringförmigen Verbindung (26) aus weniger flexiblem Material, die separat aus einem Polymer geformt und anschließend an dem Halsanlageteil (21) befestigt ist, dadurch gekennzeichnet, daß die ringförmige Verbindung (26) während des Formens mit einem Paar gegenüberliegender Lagerstifte (29) versehen wird, die nach innen in eine mittige Öffnung (28) der ringförmigen Verbindung (26) ragen, und nach dem Formen in eine Spritzform zum Formen des Halsanlageteils (21) eingeführt wird, wonach in der Spritzform zur Formung des Halsanlageteils die ringförmige Verbindung (26) mit Polymer umspritzt wird, derart, daß die ringförmige Verbindung (26) ganz bis auf das Paar gegenüberliegender Lagerstifte (29) in dem flachen folienartigen Halsanlageteil (21) eingekapselt ist.

13. Verfahren nach Anspruch 12, dadurch gekennzeichnet, daß der Formungsvorgang die Schritte der Auswahl von Polymeren aufweist, die flexibel und transparent sind, derart, daß der Halsanlageteil (21) flexibler als die ringförmige Verbindung (26) ist und die Kombination transparent ist.

14. Kombination eines Halsflansches (11) nach einem der Ansprüche 1-11 mit einem Tracheotomieschlauch (10) zur Verwendung bei einem Tracheotomievorgang im Hals eines Menschen, wobei der Tracheotomieschlauch (10) ein in den Hals eines Menschen zu positionierendes distales Ende (13), das als Durchgang (14) und Atemhilfe dient, ein außerhalb des Halses des Menschen zu positionierendes proximales Ende (15) und einen Mittelteil (16) zwischen den Enden (13, 15) aufweist, dadurch gekennzeichnet, daß der Mittelteil (16) Aussparungen (17) an daran einander gegenüberliegenden Seiten aufweist, wobei das Paar gegenüberliegender Lagerstifte (29) derart angeordnet ist, daß es zur Bewegung in die Aussparungen (17) eindrückbar ist.

## Revendications

1. Bride (11) de cou pour tube (10) de trachéostomie, destinée à positionner et supporter le tube de trachéostomie (10) lorsqu'il est introduit dans le cou (12) d'un être humain, dans lequel la bride (11) de cou possède une partie plate (21) de coopération avec le cou, en forme de feuille, constituée d'un matériau polymère souple et possédant un orifice central (25) et une interconnexion (26) d'un matériau polymère qui est moins souple que le matériau polymère de la partie (21) de coopération avec le cou, ayant un corps (27) de forme annulaire traversé par une ouverture (28) occupant un espace commun à l'orifice (25) et fixé à la partie (21) de coopération avec le cou, en étant supporté par celle-ci, l'interconnexion (26) étant destinée à être raccordée à un tube de trachéostomie (10) passant dans l'ouverture (28), caractérisée en ce que l'interconnexion (26) comporte deux broches opposées (29) de pivot dépassant vers l'intérieur dans l'ouverture (28) l'une vers l'autre et destinées à supporter de façon mobile le tube de trachéostomie (10), le corps de forme annulaire (27) étant enrobé par le polymère de la partie (21) de coopération avec le cou avec la paire de broches opposées (29) de pivot qui restent sans être enrobées.

2. Bride de cou selon la revendication 1, caractérisée en ce que l'interconnexion (26) permet un mouvement limité de pivotement du tube de trachéostomie (10) par rapport à l'ouverture (28) et pratiquement dans le plan perpendiculaire de façon générale à la feuille plate de la partie (21) de coopération avec le cou.

3. Bride de cou selon la revendication 1 ou 2, caractérisée en ce que l'interconnexion (26) a une section à gradin de manière que la paire de broches opposées (29) formant pivot soit en saillie par rapport à l'une des grandes faces du corps annulaire (27) de l'interconnexion (26).

4. Bride de cou selon l'une quelconque des revendications précédentes, caractérisée en ce que la partie (21) de coopération avec le cou et l'interconnexion (26) sont moulées sous forme de polymères différents.

5. Bride de cou selon l'une quelconque des revendications précédentes, caractérisée en ce que la partie (21) de coopération avec le cou et l'interconnexion (26) sont pratiquement transparentes.

6. Bride de cou selon la revendication 5, caractérisée en ce que la partie (21) de coopération avec le cou et l'interconnexion (26) restent transparentes après stérilisation.

7. Bride de cou selon la revendication 5 ou 6, caractérisée en ce que la partie (21) de coopération avec le cou et l'interconnexion (26) sont telles qu'elles restent imperceptiblement moins transparentes après une stérilisation par irradiation avec une dose pouvant atteindre 0,035 J/kg (3,5 rad).

8. Bride de cou selon l'une quelconque des revendications précédentes, caractérisée en ce que l'interconnexion (26) est formée d'un polymère moulé dont la dureté est supérieure à celle du polymère de la partie (21) de coopération avec le cou.

9. Bride de cou selon l'une quelconque des revendications précédentes, caractérisée en ce que la dureté de l'interconnexion (26) est une dureté Shore D de 85 selon la norme ASTM D-785 si bien que la paire de broches opposées (29) de pivot est moins flexible que la partie (21) de coopération avec le cou.

10. Bride de cou selon l'une quelconque des revendications 1 à 8, caractérisée en ce que la dureté de la paire de broches opposées (29) de pivot est une dureté Shore D égale à 85 selon la norme ASTM D-785.

11. Bride de cou selon l'une quelconque des revendications précédentes, caractérisée en ce que la dureté de la partie (21) de coopération avec le cou est une dureté Shore A comprise entre 70 et 85 selon la norme ASTM D-2240.

12. Procédé de fabrication d'une bride de cou (11) ayant une partie (21) de coopération avec le cou analogue à une feuille plate constituée d'un matériau souple et une interconnexion de forme annulaire (26) formée d'un matériau moins souple et moulée séparément en un polymère puis fixée à la partie (21) de coopération avec le cou, caractérisé en ce que l'interconnexion de forme annulaire (26) est munie, pendant le moulage, d'une paire de broches opposées (29) de pivot dépassant vers l'intérieur dans une ouverture centrale (28) de l'interconnexion de forme annulaire (26), et après moulage, elle pénètre dans un moule de moulage de la partie (21) de coopération avec le cou, puis le polymère est injecté dans le moule pour la formation de la partie (21) de coopération avec le cou autour de l'interconnexion de forme annulaire (26) d'une manière telle que l'interconnexion de forme annulaire (26) en totalité sauf au niveau des broches opposées (29) de pivot est enrobée dans la partie (21) de coopération avec le cou analogue à une feuille plate.

13. Procédé selon la revendication 12, caractérisé en ce que l'opération de moulage comporte des étapes de sélection de polymères qui sont souples et transparents afin que la partie (21) de coopération avec le cou soit plus souple que l'interconnexion de forme annulaire (26) et que la combinaison soit transparente.

14. Ensemble comprenant une bride de cou (11) selon l'une quelconque des revendications 1 à 11 et un tube de trachéostomie (10), destiné à être utilisé dans une opération de trachéostomie dans le cou d'un être humain, le tube de trachéostomie (10) ayant une extrémité distale (13) destinée à être placée dans le cou d'un être humain pour former un passage (14) et une assistance à la respiration, une extrémité proximale (15) étant destinée à être placée à l'extérieur du cou de l'être humain et une partie centrale (16) étant placée entre les extrémités (13, 15), caractérisé en ce que la partie centrale (16) comporte des cavités (17) placées sur ses côtés opposés et en face l'une de l'autre, les deux broches opposées (29) de pivot étant destinées à se déplacer dans les cavités (17).
